# EUROPEAN PATENT APPLICATION

(11) **EP 3 854 820 A1**
(43) Date of publication of application: **28.07.2021**
(21) Application number: 19861420.8
(22) Date of filing: 21.08.2019
(51) Int. Cl.: C08F 2/44, A61C 13/07, C08F 257/02, C08F 265/06, C08L 101/00

(54) **PHOTOCURABLE COMPOSITION, DENTAL PLATE LINER, AND KIT FOR PREPARING THESE**

(30) Priority: 21.09.2018 JP 2018176739
(71) Applicant: Tokuyama Dental Corporation, Tokyo 110-0016 (JP)
(72) Inventor: KINOSHITA, Masaki, Tokyo 110-0016 (JP); YAMAZAKI, Tatsuya, Tokyo 110-0016 (JP)
(74) Representative: Markfort, Iris-Anne Lucie
(86) International application number: PCT/JP2019/032694
(87) International publication number: WO 2020/059399

(57) **Abstract**

[Object] To provide a photocurable composition capable of reducing the amount of surface-unpolymerized-products of an obtained cured body and achieving a high photocuring depth, and to provide, particularly, a photocurable composition that can be suitably used in a photocuring-type denture reline material.

[Solving Means] A photocurable composition includes: a polymerizable monomer such as 2-methacryloxyethyl propionate and 1,9-nonamethylene methacrylate; resin particles such as spherical poly(ethyl methacrylate) particles; an α-diketone compound such as camphorquinone; a tertiary amine compound such as ethyl p-dimethylaminobenzoate; and an α-hydroxycarboxylic acid having an acid dissociation constant of 3.0 or more in water (at 25°C) and two or more carbonyl groups in the same molecule, such as malic acid.

## Description

### Technical Field

The present invention relates to a photocurable composition. More particularly, the present invention relates to a photocurable composition that can be suitably used as a photopolymerization-type denture reline material.

### Background Art

A denture reline material is used as a material for repairing dentures that are poorly fitted with an oral mucosa of a patient. As the denture reline material, a powder-liquid denture reline material formed of a powder material that contains resin particles as a main component and a liquid material that contains a polymerizable monomer as a main component is widely known. Such a powder-liquid denture reline material is used in the following way. A powder material and a liquid material are mixed to obtain a paste, and the paste is put on a denture and attempted to be fit with an oral mucosa before being finally cured. There are two types of powder-liquid denture reline materials, i.e., a chemical-polymerization-type one and a photopolymerization-type one, depending on the type of curing catalyst to be used.

That is, in the chemical-polymerization-type one, radicals are generated by coexistence of a chemical polymerization initiator and a polymerizable monomer and polymerization curing occurs. Usually, the polymerization curing occurs only by mixing a powder material and a liquid material. Meanwhile, in the photopolymerization-type one, polymerization curing is performed by generating radicals by applying light (hereinafter, referred to simply as "activation light") that excites a photopolymerization initiator. Usually, a paste obtained by mixing a powder material and a liquid material is put on a denture, attempted to be fitted with the inside of an oral cavity, and then finally cured by photoirradiation using a dedicated light irradiator outside the oral cavity.

A photopolymerization-type denture reline material using, as a highly-active photopolymerization initiator, a photopolymerization initiator that includes (1) α-diketone, (2) orthophosphoric acid, condensed phosphoric acid, or a non-polymerizable acidic ester thereof, and (3) a specific carbonyl-substituted aromatic amine is known (see Patent Literature 1). The final curing of such a denture reline material is usually performed using a dedicated light irradiator that uses a light source capable of applying light having a wavelength region of approximately 360 to 500 nm (main absorption region of an α-diketone compound) as activation light with a power such that the light intensity in the wavelength region is approximately 100 to 6000 mW/cm².

In the photopolymerization-type one, since a denture reline material in which the rubber-elasticity has developed is removed from the oral cavity prior to the final curing, the patient can be treated without pain even in the case of undercutting.

While the photopolymerization-type one has such an advantage, there is a problem that polymerization inhibition occurs due to oxygen in air during photocuring and an unpolymerized layer tends to be formed on the surface of a cured body because the denture reline material is removed to the outside of the oral cavity prior to the final curing. The presence of the unpolymerized layer on the surface of the denture reline material facilitates adhesion of food residues or leads to inflammation in the oral cavity due to residual monomers in some cases. Further, in the case where the unpolymerized layer is attempted to be removed by polishing or grinding in order to suppress the occurrence of such a problem, the unpolymerized layer tends to be entangled in the polishing bar, and thus, the polishing property is deteriorated in some cases. In addition, the fitting property with the oral cavity is deteriorated due to the removal of the unpolymerized layer in some cases.

As a method of preventing a layer of surface-unpolymerized-products from being formed in the photopolymerization-type one, a method of applying a polyvinyl alcohol aqueous solution to a denture reline material before being photocured (in a state in which rubber-elasticity has developed) and blocking oxygen in air that inhibits polymerization to prevent a layer of surface-unpolymerized-products from being formed (see Patent Literature 2) and a method of polymerizing the denture reline material in the state of being immersed in water or warm water (see Patent Literature 3) are known.

### Citation List

### Patent Literature

Patent Literature 1: Japanese Patent Application Laid-open No. 2009-51925
Patent Literature 2: Japanese Patent Application Laid-open No. 1983-201628
Patent Literature 3: Japanese Patent Application Laid-open No. 1987-68452

### Disclosure of Invention

### Technical Problem

In accordance with a method of applying a so-called air barrier agent for blocking contact with oxygen in air or immersing in water or the like as in the above-mentioned methods described in Patent Literatures 2 and 3, it is possible to prevent a layer of surface-unpolymerized-products from being formed. However, these operations need to be performed after achieving the fitting with the oral cavity, and it is inevitable that the operation of the operator becomes complicated. In addition, if the operator forgets to perform these operations, the effect cannot be achieved at all.

Meanwhile, it is considered that, if a very highly-active photopolymerization initiator as disclosed in Patent Literature 1 is used, a layer of surface-unpolymerized-products can be prevented from being formed. However, the formation of a layer of surface-unpolymerized-products is not particularly recognized as a problem in Patent Literature 1, and it has been unclear how much effect the photopolymerization initiator has on preventing a layer of surface-unpolymerized-products from being formed.

In this regard, when the present inventors have examined the effect, it has been found that although the effect of preventing a layer of surface-unpolymerized-products from being formed is observed to some extent, the effect is not necessarily enough, and there is room for further improvement.

It is an object of the present invention to reduce the amount of surface-unpolymerized-products of a cured body and achieve a higher photocuring depth in a photocurable composition used in a photocuring-type denture reline material and the like.

### Solution to Problem

In order to solve the above-mentioned problem (to achieve the above-mentioned object), the present inventors have intensively studied a combination of components having a high effect of preventing a layer of surface-unpolymerized-products from being formed regarding a highly-active photopolymerization initiator including a combination of α-diketone, a tertiary amine compound, and a non-polymerizable acidic compound, which is similar to that disclosed in Patent Literature 1. As a result, the present inventors have found that, in the case where a specific non-polymerizable acidic compound is used as a non-polymerizable acidic compound, reduction in the amount of surface-unpolymerized-products and improvement in the photocuring depth can be simultaneously achieved, and thus have completed the present invention.

That is, the first present invention is a photocurable composition characterized by including: a polymerizable monomer; resin particles; an α-diketone compound; a tertiary amine compound; and an α-hydroxycarboxylic acid having an acid dissociation constant of 3.0 or more in water (at 25°C) and two or more carbonyl groups in the same molecule.

The photocurable composition according to the present invention is favorably a dental photocurable composition. In particular, in the application of a denture reline material, it is favorable that the amounts of the resin particles, the α-diketone compound, the tertiary amine compound, and the α-hydroxycarboxylic acid to be blended with respect to 100 parts by mass of the polymerizable monomer are respectively 100 to 260 parts by mass of the resin particles, 0.1 to 1.0 parts by mass of the α-diketone compound, 0.1 to 2.0 parts by mass of the tertiary amine compound, and 0.005 to 0.1 parts by mass of the α-hydroxycarboxylic acid, from the point of view of the mechanical strength at the time of curing in addition to the operability at the time of mixing. Further, it is favorable to use malic acid as the α-hydroxycarboxylic acid.

The second present invention is a kit for preparing the photocurable composition according to the present invention, characterized by including: a liquid material that includes the polymerizable monomer, the tertiary amine compound, and the α-hydroxycarboxylic acid; and a powder material that includes the resin particles and the α-diketone compound.

The third present invention is a denture reline material that includes the photocurable composition according to the first present invention. Note that with regard to the third present invention, although a cured body of the photocurable composition is actually a denture reline material (material finally lined on a denture base), the composition itself prior to curing is often referred to as a denture reline material, and the composition prior to curing is referred to as a denture reline material herein.

The fourth present invention is a kit for preparing the denture reline material according to the present invention, characterized by including: a liquid material that includes the polymerizable monomer, the tertiary amine compound, and the α-hydroxycarboxylic acid; and a powder material that includes the resin particles and the α-diketone compound.

### Mode(s) for Carrying Out the Invention

The photocurable composition according to the present invention has curability higher than that of a photocurable composition using an existing photopolymerization initiator composition, and it is possible to achieve the reduction in the amount of a layer of surface-unpolymerized-products of a cured body and a higher photocuring depth. Therefore, the photocurable composition can be particularly suitably used as a denture reline material for dental use. In the denture reline material according to the present invention formed of the photocurable composition according to the present invention, it is possible to reduce the formation of a layer of surface-unpolymerized-products without the use of an air barrier agent that involves complicated operations, and thus, such operations can be omitted in some cases. Therefore, also in the case where the denture reline material is used on the premise of the use of an air barrier agent, it is possible to obtain a cured body with a less layer of surface-unpolymerized-products even when it is forgotten to use the air barrier agent due to an error or the like. In addition, without using a special chemical solution or a dedicated photopolymerization tank (for immersion in water), it is also possible to achieve a higher effect of preventing a layer of surface-unpolymerized-products from being formed, by, for example, adding a simple operation such as humidifying the atmosphere or spraying mist-like water to adhere water to the surface of the cured body.

The reason why such excellent effects can be achieved is not necessarily clear, but the present inventors speculate as follows. That is, the present inventors speculate that not only an α-hydroxycarboxylic acid having an acid dissociation constant of 3.0 or more in water (25°C) and two or more carbonyl groups in the same molecule is capable of acting as a proton donor in the composition because hydrogen at the α-position of the carboxylic acid group having electron-withdrawing property is easily abstracted, but also deactivation due to salt-forming with the tertiary amine compound hardly occurs because of the low acidity, thereby achieving high activation. Further, the present inventors speculate that the α-hydroxycarboxylic acid functions as an oxygen-blocking film by being disposed on the gas phase interface of the photocurable composition to form a water molecular film, which contributes to preventing a layer of surface-unpolymerized-products from being formed.

Hereinafter, the present invention will be described in detail. The photocurable composition according to the present invention includes: a polymerizable monomer (hereinafter, referred to also as the (a) component); resin particles (hereinafter, referred to also as the (b) component); an α-diketone compound (hereinafter, referred to also as the (c) component); a tertiary amine compound (hereinafter, referred to also as the (d) component); and an α-hydroxycarboxylic acid having an acid dissociation constant of 3.0 or more in water (at 25°C) and two or more carbonyl groups in the same molecule (hereinafter, referred to also as the (e) component). Of these components, the (a) component and the (b) component constitute the main body of a cured body after curing, and a combination of the (c) component, the (d) component, and the (e) component constitute a photopolymerization initiator.

As described above, one of the largest features of the photocurable composition according to the present invention is to use the (e) component as a non-polymerizable acidic compound in a highly-active photopolymerization initiator that includes the (c) component, the (d) component, and the non-polymerizable acidic compound, which has been known to exhibit high activity. Accordingly, the (a) component to the (d) component are not particularly different from those used in the existing photocurable composition or dental photocurable composition using the above-mentioned highly-active photopolymerization initiator, and the respective components will be described below, including the (a) component to the (d) component.

### (a) component: polymerizable monomer

As the polymerizable monomer of the (a) component, a radical polymerizable monomer can be suitably used. Examples of the radical polymerizable monomer include a known radical polymerizable monomer without any limitation. Among these, a (meth)acrylate (methacrylate or acrylate) polymerizable monomer is suitably used from the viewpoint of the mechanical strength of the obtained cured body and the dispersibility of the (b) component: resin particles in the cured body. In particular, in the case where the photocurable composition according to the present invention is a dental photocurable composition, monofunctional to tetrafunctional (meth)acrylate polymerizable monomers for dental use can be suitably used. That is, a monofunctional polymerizable monomer having one (meth)acrylate group within one molecule, a difunctional polymerizable monomer having two (meth)acrylate groups in one molecule, a trifunctional polymerizable monomer having three (meth)acrylate groups in one molecule, and a tetrafunctional polymerizable monomer having four (meth)acrylate groups in one molecule can be suitably used. These polymerizable monomers that can be suitably used will be specifically exemplified below in accordance with the above-mentioned classifications. Note that the exemplified polymerizable monomer can be used alone or a plurality of types of the polymerizable monomers can be used in combination.

### <Monofunctional (meth)acrylate polymerizable monomer>

Specific examples of the monofunctional (meth)acrylate polymerizable monomer that can be suitably used include methyl (meth)acrylate, ethyl (meth)acrylate, propyl (meth)acrylate, butyl (meth)acrylate, isopropyl (meth)acrylate, hydroxyethyl (meth)acrylate, methoxyethylene glycol (meth)acrylate, n-hexyl (meth)acrylate, cyclohexyl (meth)acrylate, tetrahydrofurfuryl (meth)acrylate, dimethylaminoethyl (meth)acrylate, n-octadecyl (meth)acrylate, n-dodecyl (meth)acrylate, n-tridecyl (meth)acrylate, 2-(meth)acryloxyethylpropionate, ethoxyethylene glycol (meth)acrylate, acetoacetoxyethyl (meth)acrylate, acetoacetoxypropyl (meth)acrylate, acetoacetoxybutyl (meta)acrylate, and diethylaminoethyl (meth)acrylate.

### <Difunctional (meth)acrylate polymerizable monomer>

Specific examples of the difunctional (meth)acrylate polymerizable monomer that can be suitably used include ethylene glycol di(meth)acrylate, diethylene glycol di(meth)acrylate, triethylene glycol di(meth)acrylate, tetraethylene glycol di(meth)acrylate, pentaethylene glycol di(meth)acrylate, hexaethylene glycol di(meth)acrylate, octaethylene glycol di(meth)acrylate, nonaglycol di(meta)acrylate, decayethyleneglycol di(meth)acrylate, undecaethylene glycol di(meth)acrylate, dodecaethylene glycol di(meth)acrylate, tridecaethylene glycol di(meth)acrylate, tetradecaethylene glycol di(meth)acrylate, pentadecaethylene glycol di(meth)acrylate, hexadecaethylene glycol di(meth)acrylate, heptadecaethylene glycol di(meth)acrylate, octadecaethylene glycol di(meth)acrylate, propylene glycol di(meth)acrylate, butylene glycol di(meth)acrylate, neopentyl glycol di(meth)acrylate, 1,3-butanediol di(meth)acrylate, 1,4-butanediol (meth)acrylate, 1,6-hexanediol d(meth)acrylate, 1,9-nonanediol di(meta)acrylate, 1,10-decanediol di(meth)acrylate, 1,12-stearyl di(meth)acrylate, 2,2-bis((meth)acryloxyphenyl)propane, 2,2-bis[4-(2-hydroxy-3-(meth)acryloxyphenyl)]propane, 2,2-bis(4-(meth)acryloxyethoxyphenyl)propane, 2,2-bis(4-(meth)acryloxydiethoxyphenyl)propane, and 2,2-bis(4-(meth)acryloxypropoxyphenyl)propane.

### <Trifunctional (meth)acrylate polymerizable monomer>

Examples of the trifunctional (meth)acrylate polymerizable monomer that can be suitably used include trimethylolpropane tri(meta)acrylate, pentaerythritol tri(meth)acrylate, and trimethylolmethane tri(meth)acrylate.

### <Tetrafunctional (meta)acrylate polymerizable monomer>

Examples of the tetrafunctional (meth)acrylate polymerizable monomer that can be suitably used include pentaerythritol tetra(meth)acrylate.

In the case where the photocurable composition according to the present invention and the dental photocurable composition are used as the denture reline material, it is favorable that the (a) polymerizable monomer contains 50 parts by mass or more, particularly 70 parts by mass or more, of a radical polymerizable monomer having a molecular weight of 150 or more and 1,000 or less, when the total amount of the (a) polymerizable monomer is 100 parts by mass, because a cured body with high polymerization activity and excellent mechanical strength can be achieved and low irritation can be achieved. It is particularly favorable that the (a) polymerizable monomer contains 50 parts by mass or more, particularly 70 parts by mass or more, of a radical polymerizable monomer having a molecular weight of 180 or more and 500 or less.

### (b) component: resin particles

Examples of the resin particles of the (b) component suitably used in the present invention include resin particles formed of polymethyl methacrylate, polyethyl methacrylate, propyl polymethacrylate, polybutyl methacrylate, polymethacrylate amyl, hexyl polymethacrylate, methyl methacrylate-ethyl methacrylate copolymer, ethylene vinyl acetate, polystyrene, styrene-butadiene copolymer, acrylonitrile-styrene copolymer, acrylonitrile-styrene-butadiene copolymer, or the like. The resin particles may be used alone or a plurality of types of the resin particles may be used in combination.

The shape of each of the (b) resin particles is not particularly limited, and may be either spherical or amorphous. Further, the particle diameter of each of the (b) resin particles is not particularly limited, and a plurality of resin particles having different average particle diameters may be used in combination.

However, in the case where the photocurable composition according to the present invention is for dental use, particularly, is a denture reline material, it is favorable to use resin particles having the average particle diameter (50% volume average particle diameter) of 1 to 300 µm measured by a laser diffraction method. Further, it is more favorable to use resin particles having the above-mentioned average particle diameter of 5 to 100 µm from the viewpoint of the familiarity of a powder material and a liquid material with each other when the powder material and the liquid material are mixed and exhibiting an appropriate increase in viscosity. In addition, from the viewpoint of solubility in the liquid material, swelling property, and mechanical strength of the obtained cured body, it is favorable to use resin particles having a weight-average molecular weight of 30,000 to 2,000,000 as determined by gel permeation chromatography (GPC), and more favorable to use resin particles having a weight-average molecular weight of 50,000 to 1,000,000.

In the photocurable composition according to the present invention, the blending ratio of the (b) resin particles is favorably 100 to 260 parts by mass with respect to 100 parts by mass of the (a) polymerizable monomer from the viewpoint of the bending strength of the obtained cured body. In particular, in the case where the photocurable composition is used for preparing a denture reline material, the blending ratio of the (b) resin particles is favorably 150 to 210 parts by mass with respect to 100 parts by mass of the (a) polymerizable monomer.

### (c) component: α-diketone compound

The α-diketone compound of the (c) component is a compound with the maximum absorbance wavelength between 350 and 700 nm and a function to grow active species effective for polymerization such as radicals by activation light. The active species usually result from energy-transfer or electron-transfer with a polymerizable monomer or another material.

Examples of the (c) α-diketone compound suitably used in the present invention include camphorquinone, benzyl, diacetyl, cyclobutenedione, camphorquinone sulfonic acids, o-benzoquinone, 1,2-cyclohexanedione, 1,2-cyclopentanedione, 2,3-pentadione, p,p'-dimethoxybenzyl, p,p'-dichlorobenzyl, acenaphthenquinone, 1,2-naphthoquinone, 2,3-naphthoquinone, 1,2-anthraquinone, 2,3-anthraquinone, 1,2-phenanthrenequinone, 2,3-phenanthrenequinone, 3,4-phenanthrenequinone, and 9,10-phenanthrenequinone. Of these, camphorquinone is particularly suitably used because of the high polymerization activity, high safety to a living body, and the like.

The blending amount of the (c) α-diketone compound in the photocurable composition according to the present invention is not particularly limited. However, the cured body tends to be soft in the case where the amount is too large, and the polymerization tends to be insufficient and sufficient mechanical strength cannot be achieved in the case where the amount is too small. For this reason, it is favorable that the blending amount is 0.1 to 1.0 parts by mass, particularly favorably 0.15 to 0.8 parts by mass, with respect to 100 parts by mass of the (a) polymerizable monomer.

### (d) component: tertiary amine compound

The tertiary amine compound of the (d) component used in the present invention is a so-called reducing agent (or electron donor) and has a polymerization-promoting function. Known compounds can be used without any limitation as long as the tertiary amine compound has such a function. Specifically, any of an aromatic tertiary amine compound having an aromatic ring in a molecule and an aliphatic tertiary amine compound having no aromatic ring in a molecule can be used. Of these, it is favorable to use the aromatic tertiary amine compound from the viewpoint of odor and the like.

Typical examples of the (d) aromatic tertiary amine compound include an aromatic tertiary amine compound represented by the following general formula (1).

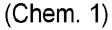

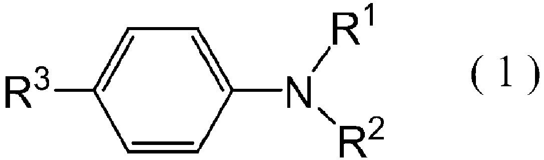

In the formula, R¹ and R² each independently represent an alkyl group and R³ represents an alkyl group, an aryl group, an alkenyl group, an alkoxy group, or an alkyloxy carbonyl group.

Examples of the compound that can be suitably used as the aromatic tertiary amine compound represented by the above-mentioned general formula (1) include methyl p-dimethylaminobenzoate, ethyl p-dimethylaminobenzoate, propyl p-dimethylaminobenzoate, amyl p-dimethylaminobenzoate, isoamyl p-dimethylaminobenzoate, ethyl p-diethylaminobenzoate, and propyl p-diethylaminobenzoate.

Further, as the (d) component, a "different aromatic tertiary amine compound" other than the aromatic tertiary amine compound represented by the above-mentioned general formula (1) can also be used. Examples of such a different aromatic tertiary amine compound include N,N-dimethylaniline, N,N-dibenzylaniline, N,N-dimethyl-p-toluidine, N,N-diethyl-p-toluidine, and N,N-di(β-hydroxyethyl)-p-toluidine.

In addition, as the tertiary amine compound of the (d) component, an aliphatic tertiary amine compound can also be used. Specific examples of the aliphatic tertiary amine compound that can be suitably used include triethanolamine, N-methyldiethanolamine, triethylamine, tributylamine, N,N-dimethylaminoethyl methacrylate, and N,N-diethylaminoethyl methacrylate.

As the (d) component, it is favorable to use an aromatic tertiary amine compound, i.e., the aromatic tertiary amine compound represented by the above-mentioned general formula (1) and/or the "different aromatic tertiary amine compound", because it exhibits low odor and high polymerization activity, maintains polymerization curability in a short time by applied light, and is capable of exhibiting high physical properties of the cured body. It is particularly favorable to use only the aromatic tertiary amine compound represented by the above-mentioned general formula (1).

In the present invention, the amount of the (d) component to be blended is favorably from 0.1 to 2 parts by mass, more favorably 0.25 to 1 parts by mass, with respect to 100 parts by mass of the polymerizable monomer of the (a) component. Further, from the viewpoint of a photocuring depth, the amount of the (d) component to be blended is favorably 30 to 330 parts by mass, more favorably 50 to 250 parts by mass, with respect to 100 parts by mass of the α-diketone compound of the component (c).

### (e) component: α-hydroxycarboxylic acid having acid dissociation constant of 3.0 or more in water (25°C) and two or more carbonyl groups in same molecule

In the photocurable composition according to the present invention, in order to prevent a layer of surface-unpolymerized-products from being formed in a cured body, it is necessary to use an α-hydroxycarboxylic acid having an acid dissociation constant of 3.0 or more in water (25°C) and two or more carbonyl groups in the same molecule, which is the (e) component, as a non-polymerizable acidic compound constituting a highly-active photopolymerization initiator together with the (c) component and the (d) component. The (e) component not only functions as a reducing agent (or electron donor) to promote polymerization similarly to the existing non-polymerizable acidic compound, but also prevents a layer of surface-unpolymerized-products from being formed presumably because water molecules contained in the air are arranged on the surface by hydroxyl groups and a water molecular film that functions as an oxygen blocking film is formed. In the case where the acid dissociation constant is less than 3.0 even if the α-hydroxycarboxylic acid has two or more carbonyl groups in the same molecule, deactivation due to salt formation with the tertiary amine compound occurs, and an effect of preventing a layer of surface-unpolymerized-products from being formed cannot be achieved.

Examples of the α-hydroxycarboxylic acid of the (e) component suitably used in the present invention include malic acid, tartaric acid, and citric acid. Of these, malic acid is favorably used from the viewpoint of solubility in a polymerizable monomer and the like.

Although the blending amount of the (e) component in the photocurable composition according to the present invention is not particularly limited, the (e) component tends to be difficult to dissolve in the polymerizable monomer if the amount is too large. Therefore, the blending amount is favorably 0.005 to 0.1 parts by mass, more favorably 0.008 to 0.08 parts by mass, with respect to 100 parts by mass of the polymerizable monomer of the (a) component.

In the photocurable composition according to the present invention, components other than the (a) to (e) components described above, such as a "different initiator component", a "different filler", water, an organic solvent, and a thickener as described below, may also be blended as long as the effect of the present invention is not impaired.

### <Different initiator>

Examples of the different initiator component include organic peroxides such as benzoyl peroxide and cumene hydroperoxide; +IV or +V vanadium compounds such as vanadium (IV) oxide acetylacetonate and bis(maltolate) oxovanadium (IV); arylborate compounds such as sodium tetraphenylboron, tetraphenyl boron triethanolamine salt, tetraphenylboron dimethyl-p-toluidine salt, sodium tetrakis(p-fluorophenyl)boron, and sodium butyltri(p-fluorophenyl)boron; acylphosphine oxides such as bis(2,4,6-trimethylbenzoyl)-phenylphosphine oxide and bis(2,6-dimethoxybenzoyl)-2,4,4-trimethylpentylphosphine oxide; benzoin alkyl ethers such as benzoin methyl ether, benzoin ethyl ether, and benzoin propyl ether; thioxanesone derivatives such as 2,4-diethoxythioxane, 2-chlorothioxansone, and methylthioxanesone; benzophenone derivatives such as benzophenone, p,p'-bis(dimethylamino)benzophenone, and p,p'-dimethoxybenzophenone.

### <Different filler>

As the different filler, inorganic particles (inorganic filler) and organic-inorganic composite particles (organic-inorganic composite filler) can be used. Specific examples of those that can be suitably used as the inorganic particles include quartz, silica, alumina, silica titania, silica zirconia, lanthanum glass, barium glass, and strontium glass. In addition, cation-eluting inorganic particles formed of hydroxides such as calcium hydroxide and strontium hydroxide; or oxides such as zinc oxide, silicate glass, and fluoroaluminosilicate glass can also be suitably used. As the organic-inorganic composite particles, particulate organic-inorganic composite particles obtained by adding the (a) polymerizable monomer to these inorganic particles in advance, making them into a paste-like state, and then polymerizing and pulverizing them can be suitably used.

There is no particular limitation on the particle diameter of each of the different fillers, and particles having an average particle diameter of 0.01 µm to 100 µm, which is generally used as a dental material, can be appropriately used depending on the purpose. Further, the refractive index of the particles is not particularly limited, and those having the refractive index in the range of 1.4 to 1.7 that general dental particles have can be used without limitation.

Note that in the case where the photocurable composition according to the present invention is used in a dental repairing material, it is favorable to use spherical inorganic particles as the different filler. By using the spherical inorganic particles, the surface smoothness of the obtained cured body increases, which can provide an excellent repairing material.

The inorganic particles (inorganic filler) described above are desirably treated with a surface treatment agent typified by a silane coupling agent in order to improve the fitting property with the polymerizable monomer and improve the mechanical strength and water resistance. The method of the surface treatment may be performed by a known method. As the silane coupling agent, methyltrimethoxysilane, methyltriethoxysilane, methyltrichlorosilane, dimethyldichlorosilane, trimethylchlorosilane, vinyltrichlorosilane, vinyltriethoxysilane, vinyltris(β-methoxyethoxy)silane, γ-methacryloyloxypropyltrimethoxysilane, γ-chloropropyltrimethoxysilane, γ-glycidoxypropyltrimethoxysilane, hexamethyldisilazane, and the like are suitably used.

Further, water, an organic solvent, a thickener, or the like can be added to the photocurable composition according to the present invention within a range that does not deteriorate the performance depending on the purpose. Examples of the organic solvent include hexane, heptane, octane, toluene, dichloromethane, methanol, ethanol, and ethyl acetate. Examples of the thickener include polymeric compounds such as polyvinylpyrrolidone, carboxymethylcellulose, and polyvinyl alcohol, and highly dispersible silica.

The photocurable composition according to the present invention can be polymerized and cured by applying light, specifically, activation light to an α-diketone compound for photosensitization. Such a curing method by photoirradiation does not particularly differ from the existing method of curing a photocurable composition including a highly-active photopolymerization initiator using an α-diketone, a tertiary amine compound, and a non-polymerizable acidic compound. In addition, a light source of visible light, such as a carbon arc, a xenon lamp, a metal halide lamp, a tungsten lamp, an LED, a halogen lamp, a helium cadmium laser, and an argon laser, is used without limitation. Since the irradiation time varies depending on the wavelength and intensity of the light source and the shape and material of the cured body, the irradiation time only needs to be determined in advance by preliminary experiments.

Since the photocurable composition according to the present invention is made difficult to form a layer of surface-unpolymerized-products during photopolymerization due to the effect of the (e) component, it is possible to achieve the effect of the present invention as described above even in the case where photopolymerization is performed by photoirradiation by the normal method as described above. Among the effects of the present invention, the effect of preventing (or reducing) the formation of a layer of surface-unpolymerized-products tends to be higher as the atmospheric humidity is higher. For example, by adding simple operations such as humidifying the atmosphere and spraying mist-like water to adhere water to the surface of the cured body, a higher effect, specifically, an effect equivalent to that of the case where an air barrier agent is applied or photopolymerization is performed by immersing in water can be achieved. Such operations of humidification or adhering water to the surface are practically useful because not only the operation itself is simple but also it is unnecessary to use a special chemical solution or a dedicated photopolymerization tank (for immersion in water). However, the present invention does not eliminate the use of an air barrier agent or photopolymerization in water, and it goes without saying that such methods can be used in combination.

The photocurable composition according to the present invention has the advantage that the curing depth at the time of polymerization and curing by photoirradiation is large and a layer of surface-unpolymerized-products is hardly formed, and can be used in various fields. The photocurable composition according to the present invention can be suitably used as a dental, particularly, photopolymerization-type denture reline material for which such characteristics are required.

As described above, the photopolymerization-type denture reline material is provided as a powder-liquid kit packed in two portions of a powder material that contains resin particles as a main component and a liquid material that contains a polymerizable monomer as a main component. Generally, the powder material and the liquid material are mixed in use to obtain a paste, put on a denture, and finally cured after being attempted to fit with the oral mucosa. In such a powder-liquid denture reline material, when the powder material and the liquid material are mixed, the polymerizable monomer that is the main component of the liquid material gradually penetrates into resin particles that are the main component of the powder material to swell the resin particles and is dissolved, resulting in paste. At this time, although the swelling and dissolution of the resin particles are small and high fluidity is observed immediately after mixing, the viscosity gradually increases as the swelling and dissolution progresses, and the mixture enters in a state being plastically deformed and then in a state of being not plastically deformed. Therefore, a user such as a dentist adjusts the mixing time to make a paste that is easy to work with, and then attempts to put the paste on a denture and make the paste fit with the oral mucosa. Assuming the actual use, the mixing time until the desired paste property is achieved is often designed to be approximately 20 seconds.

Also in the case where the photocurable composition according to the present invention is used as a denture reline material, it is favorable to make a kit of a powder material and a liquid material, and design the kit so as to have a desired pasting property in the mixing time of approximately 20 seconds. For this reason, in the case of a kit packed in two portions, it is favorable to make a kit including a liquid material that contains the (a) component, the (d) component, and the (e) component, and a powder material that contains the (b) component and the (c) component. The reason why the (e) component is blended into the liquid material is that the (e) component is often a solid at ordinary temperature, it takes a long time to dissolve, and it becomes difficult to adjust the paste property by the mixing time in the case where the (e) component is blended into the powder material.

The mixing ratio of the powder material and the liquid material is not particularly limited, and only needs to be appropriately determined in consideration of the content of the components contained in the respective members and the paste property at the time of mixing the powder material and the liquid material described above. Generally, a ratio of the powder material (g)/liquid material (ml) = 1/1 to 3/1 is favorable, and a ratio of the powder material (g)/liquid material (ml) = 1.5/1 to 2.5/1 is more favorable. Note that the (a) to (e) components only need to be blended into the powder material or the liquid material so as to have a suitable blending amount as described above when the powder material and the liquid material are mixed in the above-mentioned ratio.

Further, a pigment, a dye, and an ultraviolet-ray absorbent may be blended in addition to the respective components described above in order to match with the color tone of the oral mucosa and suppressing discoloration due to ultraviolet rays. Further, if necessary, a known additive material to be blended as a denture reline material may be blended within a range that does not affect the effect of the present invention.

The powder material and the liquid material may be produced in accordance with a known production method without any particular limitation as the method of producing the powder material and the liquid material. Specifically, each of the predetermined blending components may be weighed and mixed until a uniform property is achieved. There is no particular limitation on the apparatus that can be used for mixing, and known ones can be used. For example, the powder material and the liquid material can be produced by being uniformly mixed by a swinging mixer and a stirrer blade, respectively. Further, the produced powder material and the produced liquid material only need to be preserved in a container, and may be preserved in an arbitrary amount by subdividing them into an arbitrary amount.

As a method of using a powder-liquid denture reline material that includes the powder material and the liquid material obtained as described above, the powder material and the liquid material only need to be mixed with each other and used as appropriate in accordance with the respective forms. As an example thereof, a desired amount of the liquid material and a desired amount of the powder material are weighed into a rubber cup or the like immediately before use, and the mixture is kneaded using a kneading rod, a spatula, or the like until a uniform paste is obtained. The paste having a moderate property by the kneading is put on a denture that has become poorly fitted, attempted to fit with an oral cavity, and then polymerized and cured by applying activation light thereto using a dedicated light irradiator, thereby preparing a denture reline material.

### (Example)

Hereinafter, in order to specifically describe the present invention, Examples and Comparative Examples will be described, but the present invention is not limited in any way by these.

First, the names, characteristics, abbreviations (if abbreviations are used), and the like of materials and the like used for preparing samples according to the respective Examples and Comparative Examples will be described for each component.

### (a) component: polymerizable monomer

▪HPr: 2-methacryloxyethyl propionate (monofunctional polymerizable monomer, a molecular weight of 186)
▪ ND: 1,9-nonamethylenediol dimethacrylate (difunctional polymerizable monomer, a molecular weight of 296)
▪TT: Trimethylolpropane trimethacrylate (trifunctional polymerizable monomer, a molecular weight of 338)
▪PT: pentaerythritol tetramethacrylate (tetrafunctional polymerizable monomer, a molecular weight of 367)

### (b) component: resin particles

▪PEMA: spherical poly(ethyl methacrylate) particles (average particle diameter of 35 µm, a weight average molecular weight of 500,000)
▪PS: spherical polystyrene particles (non-crosslinked, an average particle diameter of 30 µm, a weight average molecular weight of 400,000)

### (c) component: α-diketone compound

▪Camphorquinone
▪Benzyl

### (d) component: tertiary amine compound

▪DMBE: ethyl p-dimethylaminobenzoate
▪MDEOA: N-methyldiethanolamine

### (e) component: non-polymerizable acidic compound (numerical values in parentheses indicate acid dissociation constants in water at 25°C)

▪Malic acid (3.4)
▪Tartaric acid (3.0)

### (e') component: non-polymerizable acidic compound that does not correspond to the (e) component (numerical values in parentheses indicate acid dissociation constants in water at 25°C)

▪Orthophosphoric acid (2.1)

### (f) component: other components

▪Dibutylhydroxytoluene
▪Organic pigment
▪10% polyvinyl alcohol aqueous solution

Next, the sample preparation method in each of Examples and Comparative Examples and the method of measuring evaluation items for the sample will be described.

### (1) Method of measuring amount of surface-unpolymerized-products (unpolymerized layer) of cured body

A powder material and a liquid material were placed in a rubber cup at a ratio of the powder material (g)/liquid material (ml) = 1.8/1 and mixed for 20 seconds. The mixed photocurable composition (photocurable composition obtained by mixing the powder material and the liquid material) was poured into a mold made of polytetrafluoroethylene of 20 x 20 x 1 mm, and a cured body was prepared by applying activation light (optical power density of 100 mW/cm²) having a wavelength of 465 to 475 nm for 5 minutes using an optical polymerization apparatus α light V for dental engineering (manufactured by MORITA Co., Ltd.) with one side exposed to the atmosphere. After measuring the thickness of the obtained cured body by 5 points, the cured body was immersed in ethanol for 1 minute, and the unpolymerized layer was scraped with a metallic spatula. The thickness of the cured body from which the layer of surface-unpolymerized-products was removed was measured, and the difference between the average thickness of the cured body prior to immersion in ethanol and the average thickness of the cured body after removal with a metal spatula was taken as the thickness of the layer of surface-unpolymerized-products. Note that in Example 39 and Comparative Example 5, the photocurable composition was once immersed in ion-exchanged water to adhere water to the surface thereof prior to photoirradiation with the optical polymerization apparatus α light V for dental engineering, and then taken out into the gas phase to perform photocuring thereon. Other operations were performed in the way as described above in the examination. Further, in Comparative Example 6, a 10% polyvinyl alcohol aqueous solution was applied to the photocurable composition prior to photoirradiation with the optical polymerization apparatus α light V for dental engineering to prepare a cured body. Other operations were performed in the way as described above in the examination.

### (2) Measurement of photocuring depth of cured body

A powder material and a liquid material were placed in a rubber cup at a ratio of the powder material (g)/liquid material (ml) = 1.8/1 and mixed for 20 seconds. A black rubber tube having an inner diameter of 4 mmϕ was filled with the kneaded photocurable composition (photocurable composition obtained by mixing the powder material and the liquid material), and both sides thereof were pressed against each other with a polypropylene film. After that, the photocurable composition was immediately irradiated with activation light (optical power density of 700 mW/cm²) having a wavelength of 400 to 520 nm for 60 seconds using a dental light irradiator Tokuso Power Light (manufactured by Tokuyama Dental Co., Ltd.) and cured. The sample was taken out from the black rubber tube, the uncured portion was removed, and then the length of the cured portion was measured by a caliper to take it as a photocuring depth. Note that in Example 39 and Comparative Example 5, the photocurable composition was once immersed in ion-exchanged water prior to pressuring with a polypropylene film, and then taken out into the gas phase to perform photocuring thereon. Further, in Comparative Example 6, a 10% polyvinyl alcohol aqueous solution was applied to the photocurable composition prior to pressuring with a polypropylene film and then the photocurable composition was cured. Other operations were performed in the way as described above in the examination.

### (3) Measurement of three-point bending strength of cured body

The powder material and the liquid material were placed in a rubber cup at a ratio of the powder material (g)/liquid material (ml) = 1.8/1 and mixed for 20 seconds. The mixed photocurable composition (photocurable composition obtained by mixing the powder material and the liquid material) was poured into a mold made of polytetrafluoroethylene of 30 x 30 x 2 mm, and a cured body was prepared by applying activation light (optical power density of 100 mW/cm²) having a wavelength of 465 to 475 nm for 5 minutes using an optical polymerization apparatus α light V for dental engineering (manufactured by MORITA Co., Ltd.) with both sides thereof pressed against each other with a polypropylene film. Subsequently, the cured body was polished with #800 and #1500 water-resistant abrasive papers, and then cut into a prismatic test piece of 4 x 30 x 2 mm. The test piece was mounted on a testing machine (Autograph AG5000D manufactured by Shimadzu Corporation), and the three-point bending strength was measured under the conditions of the distance between the fulcrums of 20 mm and the crosshead speed of 1 mm/min. Note that in Example 39 and Comparative Example 5, the photocurable composition was once immersed in ion-exchanged water prior to pressuring with a polypropylene film, and then taken out into the gas phase to perform photocuring thereon. Further, in Comparative Example 6, a 10% polyvinyl alcohol aqueous solution was applied to the photocurable composition prior to pressuring with the polypropylene film, and then cured. Other operations were performed in the way as described above in the examination.

### Example 1

As a powder material, the (b) resin particles: PEMA (180 parts by mass) and the (c) α-diketone compound: camphorquinone (0.3 parts by mass) were mixed. Meanwhile, as a liquid material, the (a) monofunctional polymerizable monomer: HPr (60 parts by mass), the difunctional polymerizable monomer: ND (40 parts by mass), the (d) tertiary amine compound: DMBE (0.5 parts by mass), and the (e) α-hydroxycarboxylic acid having an acid dissociation constant of 3.0 or more in water (25°C) and two or more carbonyl groups in the same molecule: malic acid were mixed. The obtained powder material and liquid material were used to prepare a cured body in accordance with the methods described in the above-mentioned evaluation methods (1) to (3), and the amount of surface-unpolymerized-products, the photocuring depth, and the three-point bending strength of the obtained cured body were evaluated.

The compositions of the powder material and the liquid material are shown in Table 1 and Table 2. Further, the evaluation results are shown in Table 4.

### Examples 2 to 38

A powder-liquid type photocurable composition (denture reline material composition) was prepared by a method similar to that in Example 1 except that the compositions of the powder material and the liquid material were changed as shown in Table 1 and Table 2. The obtained photocurable composition was used to prepare a cured body by a method similar to that in Example 1, and the obtained cured body was evaluated by a method similar to that in Example 1. The evaluation results are shown in Table 4.

### Example 39

A powder-liquid type photocurable composition (denture reline material composition) was prepared by a method similar to that in Example 1 except that the compositions of the powder material and the liquid material were changed as shown in Table 1 and Table 2. The obtained powder material and the obtained liquid material were used to prepare a cured body in accordance with the methods described in the above-mentioned evaluation methods (1) to (3), and the obtained cured body was evaluated by a method similar to that in Example 1. The evaluation results are shown in Table 4.

### Comparative Examples 1 to 4

A powder-liquid type photocurable composition (denture reline material composition) was prepared by a method similar to that in Example 1 except that the compositions of the powder material and the liquid material were changed as shown in Table 1 and Table 3. The obtained photocurable composition was used to prepare a cured body by a method similar to that in Example 1, and the obtained cured body was evaluated by a method similar to that in Example 1. The evaluation results are shown in Table 4.

### Comparative Examples 5 and 6

A powder-liquid type photocurable composition (denture reline material composition) was prepared by a method similar to that in Example 1 except that the compositions of the powder material and the liquid material were changed as shown in Table 1 and Table 3. The obtained powder material and the obtained liquid material were used to prepare a cured body in accordance with the methods described in the above-mentioned evaluation methods (1) to (3), and the obtained cured body was evaluated by a method similar to that in Example 1. The evaluation results are shown in Table 4.

**(Table 1)**

| | Powder material | | | | | |
|---|---|---|---|---|---|---|
| | (b)Resin particles | | (c)α-diketone compound | | (e)Non-polymerizable acidic compound | |
| | PEMA | PS | Camphorquinone | Benzyl | Malic acid | Tartaric acid |
| Example 1-8 | 180 | | 0.3 | | | |
| Example 9 | 180 | | 0,05 | | | |
| Example 10 | 180 | | 0.15 | | | |
| Example 11 | 180 | | 0.2 | | | |
| Example 12 | 180 | | 0.7 | | | |
| Example 13 | 180 | | 0.9 | | | |
| Example 14 | 180 | | 1. 5 | | | |
| Example 15-16 | 180 | | | 0.3 | | |
| Example 17-28 | 180 | | 0.3 | | | |
| Example 29 | 60 | | 0.3 | | | |
| Example 30 | 110 | | 0.3 | | | |
| Example 31 | 150 | | 0.3 | | | |
| Example 32 | 210 | | 0.3 | | | |
| Example 33 | 250 | | 0.3 | | | |
| Example 34 | 310 | | 0.3 | | | |
| Example 35-36 | | 180 | 0.3 | | | |
| Example 37 | 180 | | 0.3 | | 0.03 | |
| Example 38 | 180 | | 0.3 | | | 0.03 |
| Example 39 | 180 | | 0.3 | | | |
| Comparative Example 1-2 | 180 | | 0.3 | | | |
| Comparative Example 3 | 180 | | | | | |
| Comparative Example 4-6 | 180 | | 0.3 | | | |

**(Table 2)**

| | Liquid material | | | | | | | |
|---|---|---|---|---|---|---|---|---|
| | (a)Polymerizable monomer | | | | (d)Tertiary amine compound | | (e)Non-polymerizable acidic compound | |
| | HPr | ND | TT | PT | DMBE | MDEOA | Malic acid | Tartaric acid |
| Example 1 | 60 | 40 | | | 0.5 | | 0.03 | |
| Example 2 | 60 | 40 | | | 0.5 | | | 0. 03 |
| Example 3 | 60 | 40 | | | 0.5 | | 0.003 | |
| Example 4 | 60 | 40 | | | 0.5 | | 0.005 | |
| Example 5 | 60 | 40 | | | 0.5 | | 0.01 | |
| Example 6 | 60 | 40 | | | 0.5 | | 0.07 | |
| Example 7 | 60 | 40 | | | 0.5 | | 0.09 | |
| Example 8 | 60 | 40 | | | | | 0.2 | |
| Example 9-15 | 60 | 40 | | | 0.5 | | 0.03 | |
| Example 16 | 60 | 40 | | | 0.5 | | | 0.03 |
| Example 17 | 60 | 40 | | | 0.05 | | 0.03 | |
| Example 18 | 60 | 40 | | | 0.2 | | 0.03 | |
| Example 19 | 60 | 40 | | | 0.3 | | 0.03 | |
| Example 20 | 60 | 40 | | | 0.8 | | 0.03 | |
| Example 21 | 60 | 40 | | | 1.8 | | 0.03 | |
| Example 22 | 60 | 40 | | | 3.0 | | 0.03 | |
| Example 23 | 60 | 40 | | | | 0.5 | 0.03 | |
| Example 24 | 60 | 40 | | | | 0. 5 | | 0. 03 |
| Example 25 | 57 | 38 | 5 | | 0.5 | | | |
| Example 26 | 51 | 34 | 15 | | 0.5 | | 0.03 | |
| Example 27 | 57 | 38 | | 5 | 0.5 | | 0.03 | |
| Example 28 | 51 | 34 | | 15 | 0.5 | | 0.03 | |
| Example 29-35 | 60 | 40 | | | 0.5 | | 0.03 | |
| Example 36 | 60 | 40 | | | 0.5 | | | 0.03 |
| Example 37-38 | 60 | 40 | | | 0.5 | | | |
| Example 39 | 60 | 40 | | | 0.5 | | 0.03 | |

**(Table 3)**

| | Liquid material | | | | |
|---|---|---|---|---|---|
| | (a)Polymerizable monomer | | (d)Tertiary amine compound | Non-polymerizable acidic compound | |
| | | | | (e) component | (e') component |
| | HPr | IND | DMBE | Malic acid | Orthophosphoric acid |
| Comparative Example 1 | 60 | 40 | 0.5 | | 0.03 |
| Comparative Example 2 | 60 | 40 | 0.5 | | |
| Comparative Example 3 | 60 | 40 | 0.5 | 0.03 | |
| Comparative Example 4 | 60 | 40 | | 0.03 | |
| Comparative Example 5 | 60 | 40 | 0.5 | | |
| Comparative Example 6 | 60 | 40 | 0.5 | | |

**(Table 4)**

| | Thickness of surface-unpolymerized-products/µm | Photocuring depth/mm | Bending strength/MPa |
|---|---|---|---|
| Example 1 | 41 | 4.52 | 66.4 |
| Example 2 | 48 | 4.37 | 64.0 |
| Example 3 | 59 | 3.74 | 59.4 |
| Example 4 | 54 | 3.87 | 60.5 |
| Example 5 | 48 | 4.14 | 63.6 |
| Example 6 | 42 | 4.53 | 66.0 |
| Example 7 | 40 | 4.55 | 66. |
| Example 8 | 41 | 4.52 | 66.0 |
| Example 9 | 60 | 3.71 | 58.6 |
| Example 10 | 55 | 4.00 | 59.6 |
| Example 11 | 47 | 4.38 | 63.9 |
| Example 12 | 43 | 4.55 | 65.2 |
| Example 13 | 49 | 4.18 | 60.7 |
| Example 14 | 58 | 3.87 | 58.3 |
| Example 15 | 45 | 4.23 | 61.3 |
| Example 16 | 50 | 4.16 | 60.5 |
| Example 17 | 60 | 3.89 | 58.5 |
| Example 18 | 56 | 4.18 | 60.8 |
| Example 19 | 49 | 4.30 | 62.9 |
| Example 20 | 41 | 4.60 | 66.5 |
| Example 21 | 45 | 4.36 | 65.7 |
| Example 22 | 54 | 3.91 | 61.1 |
| Example 23 | 49 | 4.10 | 62.1 |

| | Thickness of surface-unpolymerized-products /µm | Photocuring depth /mm | Bending strength /MPa |
|---|---|---|---|
| Example 24 | 56 | 3.99 | 60.6 |
| Example 25 | 42 | 4.46 | 65.8 |
| Example 26 | 44 | 4.33 | 64.1 |
| Example 27 | 44 | 4.39 | 66.0 |
| Example 28 | 43 | 4.17 | 62.4 |
| Example 29 | 60 | 4.71 | 60.4 |
| Example 30 | 52 | 4.66 | 64.5 |
| Example 31 | 46 | 4.51 | 64.4 |
| Example 32 | 42 | 4.37 | 63.2 |
| Example 33 | 54 | 4.12 | 61.1 |
| Example 34 | 61 | 3.81 | 58.6 |
| Example 35 | 45 | 4.16 | 67.8 |
| Example 36 | 52 | 4.01 | 65.1 |
| Example 37 | 56 | 3.91 | 58.7 |
| Example 38 | 58 | 3.84 | 58.3 |
| Example 39 | 25 | 4.50 | 65.5 |
| Comparative Example 1 | 62 | 3.66 | 58.12 |
| Comparative Example 2 | 78 | 3.02 | 53.3 |
| Comparative Example 3 | | Not cured | |
| Comparative Example 4 | 134 | 0.82 | 31.2 |
| Comparative Example 5 | 76 | 2.95 | 51.1 |
| Comparative Example 6 | 22 | 3.10 | 55.2 |

In Examples 1 to 39, the respective components were blended to satisfy the configuration shown in the present invention. In all cases, the amount of surface-unpolymerized-products was small and a high photocuring depth was obtained. Further, in Example 39, water is adhered to the surface of the photocurable composition (denture reline material composition) and then photocuring is performed. In this case, the amount of surface-unpolymerized-products can be further reduced.

Meanwhile, in Comparative Example 1, a non-polymerizable acidic compound other than the (e) component is blended as the (E) non-polymerizable acidic compound. In this case, the amount of surface-unpolymerized-products was higher and the photocuring depth was inferior than that in the case where the (e) compound was blended.

In Comparative Example 2, the (e) component was not blended. In this case, the amount of surface-unpolymerized-products increased and a deep photocuring depth could not be obtained as well. In Comparative Example 3, the (c) α-diketone compound was not blended and it was not cured. Further, in Comparative Example 4, the tertiary amine compound was not blended. In this case, the amount of surface-unpolymerized-products increased and a deep photocuring depth could not be obtained.

In Comparative Example 5, the surface of the photocurable composition (denture reline material composition) in which the (e) component was not blended was photo-cured under wet conditions. In this case, the amount of surface-unpolymerized-products increased and a deep photocuring depth could not be obtained similarly to Comparative Example 2. From this fact, it can be seen that, when the (e) component is not blended, the amount of surface-unpolymerized-products cannot be reduced even if water is adhered to the surface.

In Comparative Example 6, a 10% polyvinyl alcohol aqueous solution was applied as an air barrier agent at the time of preparing a cured body without blending the (e) component. Although the amount of surface-unpolymerized-products could be reduced, a deep photocuring depth could not be obtained.

## Claims

1. A photocurable composition **characterized by** comprising:
a polymerizable monomer;
resin particles;
an α-diketone compound;
a tertiary amine compound; and
an α-hydroxycarboxylic acid having an acid dissociation constant of 3.0 or more in water (at 25°C) and two or more carbonyl groups in the same molecule.

2. The photocurable composition according to claim 1, wherein
amounts of the resin particles, the α-diketone compound, the tertiary amine compound, and the α-hydroxycarboxylic acid to be blended with respect to 100 parts by mass of the polymerizable monomer are respectively 100 to 260 parts by mass of the resin particles, 0.1 to 1.0 parts by mass of the α-diketone compound, 0.1 to 2.0 parts by mass of the tertiary amine compound, and 0.005 to 0.1 parts by mass of the α-hydroxycarboxylic acid.

3. The photocurable composition according to claim 1 or 2, wherein
malic acid is used as the α-hydroxycarboxylic acid.

4. A kit for preparing the photocurable composition according to any one of claim 1 to 3, **characterized by** comprising:
a liquid material that includes the polymerizable monomer, the tertiary amine compound, and the α-hydroxycarboxylic acid; and
a powder material that includes the resin particles and the α-diketone compound.

5. A denture reline material comprising the photocurable composition according to any one of claims 1 to 3.

6. A kit for preparing the denture reline material according to claim 5, **characterized by** comprising:
a liquid material that includes the polymerizable monomer, the tertiary amine compound, and the α-hydroxycarboxylic acid; and
a powder material that includes the resin particles and the α-diketone compound.

## Amended claims

### Amended claims under Art. 19.1 PCT

1. A kit for preparing a photocurable composition that contains an (a) component including a polymerizable monomer, a (b) component including resin particles having a weight-average molecular weight of 30,000 to 2,000,000 as determined by gel permeation chromatography, a (c) component including an α-diketone compound, a (d) component including a tertiary amine compound, and an (e) component including an α-hydroxycarboxylic acid having an acid dissociation constant of 3.0 or more in water (at 25°C) and two or more carbonyl groups in the same molecule,
**characterized by** comprising:
a liquid material that contains the (a) component and the (e) component; and
a powder material that contains the (b) component, wherein
each of the (c) component and the (d) component is contained in the liquid material or the powder material.

2. The kit according to claim 1, wherein
the (d) component is contained in the liquid material, and
the (c) component is contained in the powder material.

3. The kit according to claim 1 or 2, wherein
amounts of the (b) component: the resin particles, the (c) component: the α-diketone compound, the (d) component: the tertiary amine compound, and the (e) component: the α-hydroxycarboxylic acid to be blended with respect to 100 parts by mass of the (a) component: the polymerizable monomer are respectively 100 to 260 parts by mass of the (b) component, 0.1 to 1.0 parts by mass of the (c) component, 0.1 to 2.0 parts by mass of the (d) component, and 0.005 to 0.1 parts by mass of the (e) component.

4. The kit according to any one of claims 1 to 3, wherein
malic acid is used as the (e) component: the α-hydroxycarboxylic acid.

5. A kit for preparing a denture reline material, which includes a photocurable composition that contains an (a) component including a polymerizable monomer, a (b) component including resin particles having a weight-average molecular weight of 30,000 to 2,000,000 as determined by gel permeation chromatography, a (c) component including an α-diketone compound, a (d) component including a tertiary amine compound, and an (e) component including an α-hydroxycarboxylic acid having an acid dissociation constant of 3.0 or more in water (at 25°C) and two or more carbonyl groups in the same molecule, **characterized by** comprising:
a liquid material that contains the (a) component and the (e) component; and
a powder material that contains the (b) component, wherein
each of the (c) component and the (d) component is contained in the liquid material or the powder material.

6. The kit according to claim 5, wherein
the (d) component is contained in the liquid material, and
the (c) component is contained in the powder material.
